(19) Europäisches Patentamt
European Patent Office
Office européen des brevets

(11) Publication number: **0 101 254**
**A2**

# (12) EUROPEAN PATENT APPLICATION

(21) Application number: 83304498.5

(22) Date of filing: 04.08.83

(51) Int. Cl.³: **C 07 C 29/76**

(30) Priority: 05.08.82 GB 8222621

(43) Date of publication of application: 22.02.84 Bulletin 84/8

(84) Designated Contracting States: BE DE FR GB IT NL

(71) Applicant: The British Petroleum Company p.l.c.
Britannic House Moor Lane
London EC2Y 9BU(GB)

(72) Inventor: Groszek, Aleksander Jerzy
The British Petroleum Company p.l.c. Chertsey Road
Sunbury-on-Thames Middlesex TW16 7LN(GB)

(74) Representative: Harry, John et al,
BP INTERNATIONAL LIMITED Patents and Licensing Division Chertsey Road
Sunbury-on-Thames Middlesex TW16 7LN(GB)

## (54) Process for separating ethanol from dilute aqueous solutions thereof.

(57) Ethanol is separated from dilute aqueous solutions thereof by bringing the aqueous solution into contact with a crystalline aluminosilicate zeolite which is a high silica zeolite having a silica to alumina molar ratio in the range from 12:1 to 300:1, preferably from 40:1 to 60:1.

EP 0 101 254 A2

PROCESS FOR SEPARATING ETHANOL FROM DILUTE AQUEOUS SOLUTIONS THEREOF

The present invention relates to a process for separating ethanol from dilute aqueous solutions thereof.

In any process for separating components of a mixture by adsorption the adsorbent should possess the following properties:

1. A high capacity for preferential adsorption,
2. A rapid rate of adsorption (enabling the adsorbent to adsorb a significant amount of the desired component in a short time from a mixture with which it is contacted), and
3. A high selectivity for the desired component so that effective separation of the component from mixtures in which its concentration is low can be effected.

A class of adsorbents which possess these properties is the crystalline aluminosilicates, the best known of which are the zeolites. The crystalline aluminosilicates function as "molecular sieves", that is they contain pores having cross-sectional diameters which will accept certain molecules in a mixture of molecules of specific size and shape while rejecting others.

In the context of concentrating ethanol from fermentation beers, Hartline in an article entitled "Lowering the Cost of Alcohol", published in Science, Vol 206, 41-42 (1979) refers to experiments using zeolites as molecular sieves to selectively adsorb water from aqueous ethanol, thereby increasing the concentration of the residual solution. It is stated that a promising candidate for such a process is clinoptilolite, which is a naturally occurring zeolite, having a low silica to alumina ratio.

It is also known from US Patents Nos 4061724 and 4073865 that silicalite and similar crystalline silica polymorphs are capable of removing small molecule organic compounds, such as methanol, n-butanol, methyl cellosolve and phenol, from dilute aqueous solutions. Silicalite is to be distinguished from aluminosilicalite zeolites in that it has no ion-exchange capacity, a property which in USP 4061724 is said to influence to a greater or lesser degree the hydrophobic and/or pore size of aluminosilicates rendering them less suitable for the selective removal of organics from waste water streams containing a source of cations.

We have now found that synthetic crystalline aluminosilicates having a high silica to alumina molar ratio, that is a silica to alumina molar ratio in the range from 12:1 to 300:1 can selectively remove ethanol from aqueous solutions thereof.

Accordingly, the present invention is a process for the separation of ethanol from an aqueous solution thereof which comprises bringing the aqueous solution into contact with a synthetic crystalline aluminosilicate zeolite which is a high silica zeolite wherein the molar ratio of silica to alumina is in the range from 12:1 to 300:1.

The process of the present invention is most advantageously applied to relatively dilute aqueous solutions of ethanol for example solutions containing less than 70% by weight of ethanol, more preferably less than 10% by weight of ethanol, eg 2 to 8% by weight of ethanol.

The zeolites used in the process of the present invention are synthetic high silica zeolites, ie zeolites which have a significantly higher silica to alumina molar ratio than the zeolites which have conventionally been used as molecular sieves. The silica to alumina molar ratio of the zeolites of the present invention lies between 12:1 to 300:1 preferably from 40:1 to 60:1.

Various classes of high silica zeolites differing in their crystal structure are known.

Thus one class of high silica zeolite is the class known in the IUPAC terminology as the MEL structure-type zeolites. The zeolites

and processes for their production are disclosed in "Atlas of Zeolite Structure Types", by Meier et al published by the Structure Commission of the International Zeolite Association (1978). Another class of high silica zeolites are Theta-1 zeolites which are disclosed in European patent specification EP 57049. It is preferred to use the MFI-structure type zeolites.

MFI-type crystalline aluminosilicate zeolites may suitably be prepared by crystallisation for a time greater than 0.5 hr at a temperature in the range from 80 to 220°C, preferably from 120 to 175°C, from a gel comprising (i) a source of silica ($SiO_2$), (ii) a source of alumina ($Al_2O_3$), (iii) a mineralising agent selected from oxides and salts of alkali and alkaline earth metals ($X_{2/b}O$ wherein b is the valency of X), (v) an organic base (B), as hereinafter defined, and (vi) water and/or alcohol (ROH).

The term organic base as used hereinbefore means any organic material for which $-\log_{10}K$ for the reaction:

$$B + H_2O \rightleftharpoons BO^+ + OH^-$$

is less than 7, where $K = [BH^+][OH^-]/[B]$

Suitable organic bases include:

(i) tetrahydrocarbylammonium salts of the formula:

$R^1R^2R^3R^4N^+X^-$

wherein $R^1$, $R^2$, $R^3$ and $R^4$ are independently either aryl, substituted aryl, alkyl, substituted alkyl, cycloalkyl, substituted cycloalkyl or hydrogen and $X^-$ is an inorganic anion. Preferably $R^1$, $R^2$, $R^3$ and $R^4$ are alkyl groups containing from 1 to 5 carbon atoms. Preferably $X^-$ is a halide or hydroxide ion. Suitable tetraalkylammonium salts include tetraethylammonium hydroxide and tetrapropylammonium hydroxide. Alternatively, the precursors of tetraalkylammonium compounds may be used.

(ii) amines having the formula:

either $R^1R^2R^3N$, or

$R^1R^2N(CH_2)_xNR^3R^4$ wherein x is an integer in the range 1 to 20, or $R^1R^2N(CH_2)_yN(CH_2)_zNR^3R^4$ wherein y and z are integers in the range 1 to 20.

Suitable amines include $C_1$ to $C_9$ primary monoalkyl amines, such as n-ethylamine, n-propylamine and n-butylamine.

(iii) Mono-, di- and tri-alkanolamines such as monoethanolamine, diethanolamine and triethanolamine, or their precursors in the form of an alkylene oxide and ammonia.

Suitable sources of silica include, for example, sodium silicate, silica hydrosol, silica gel, silica sol and silicic acid. The preferred source of silica is an aqueous colloidal dispersion of silica particles. A suitable commercially available source of silica is LUDOX* Colloidal Silica manufactured by DuPont (* Registered Trade Mark).

Suitable sources of alumina include, for example, sodium aluminate, aluminium sulphate and alumina. The preferred source of alumina is sodium aluminate prepared by dissolving particulate alumina in excess sodium hydroxide solution.

The preferred mineralising agents are alkali and alkaline earth metal hydroxides and halides, such as for example, lithium hydroxide, sodium hydroxide, potassium hydroxide, potassium bromide and calcium bromide. The preferred mineralising agent is sodium hydroxide.

It will be appreciated that each source of silica, alumina, alkali or alkaline earth metal can be supplied by one or more initial reactants and then mixed together in any order. For example, sodium silicate is a source of both alumina and silica and an aluminosilicate is a source of both alumina and silica. Thus, the source of alumina and the source of silica may be supplied in whole or in part by an aluminosilicate, which may be either crystalline or amorphous.

Specific MFI-type zeolites and processes for their production utilising organic bases are described in US Patent Nos 3702886; 3709979, 4205053, 4116099, 4139600 and 4151189; UK Patent Nos 1365318 and 1567948 and European patent application publication Nos 2899 and 2900, for example.

Alternatively, the organic base may be replaced by an inorganic source of ammonium ions as described in our European patent

application publication No 30811, US Patent No 4119556 and UK application publication No 2018232A. It is preferred to employ an MFI-type crystalline aluminosilicate crystallised from a gel containing an inorganic source of ammonium ions, for example aqueous ammonia.

The cations normally associated with the zeolites, for example alkali metal and/or alkaline earth metal ions, organic nitrogen ions and ammonium ions may suitably be exchanged with other cations, for example hydrogen ions, using conventional techniques. Furthermore, it is desirable to calcine the zeolite, suitably by heating, for example in a stream of air, at a temperature in the range from 400 to 600°C for at least 0.5 hour.

The process of the present invention may be operated in the liquid phase or the gaseous phase.

The separated ethanol may be recovered from the zeolite by contact with a suitable desorbent which displaces the ethanol from the zeolite, or by heating the zeolite. When the ethanol is removed by heating, it will only be necessary to provide sufficient heat to evaporate the ethanol. This will be much less than the heat required to evaporate a mixture of water and ethanol by distillation.

The zeolite may be employed in the form of a dense compact fixed bed which is alternately contacted with the feed mixture and desorbent materials. In the simplest embodiment of the invention the zeolite is employed in the form of a single static bed in which case the process is only semi-continuous. In an alternative embodiment, two or more static beds are employed, the feed mixture being passed through one or more zeolite beds at the same time as the desorbent is passed through one or more other beds.

It is preferred to employ the zeolite in the form of a countercurrent moving bed or in a simulated moving bed countercurrent flow system, the principle of which is described in US Patent No 2985589.

The process of the present invention will now be illustrated by reference to the following Examples. In the Examples the following apparatus, method and adsorbents were employed.

The apparatus used in the Examples was a commercially available flow calorimeter manufactured by Microscal Limited and fitted with syringe micropumps manufactured under the trade name PERFUSOR IV by B Brown Melsungen AG.

The adsorbents used in the experiments were saturated with triply distilled water. Adsorption experiments were carried out as described in Groszek, A.J., Proc. Roy. Soc. A, 1970, 314, 473-498. A 1% wt/wt solution of ethanol in water prepared from absolute ethanol refractive index 1.361 at 20°C was percolated through the adsorbents at the rate of 0.05ml per minute and the temperature of the solution was maintained at 22°C ± 1°C.

The adsorbents were used without any previous degassing treatments i.e. as taken out of bottles where they were stored under air. The rises of temperature taking place in the adsorbent through which solutions were percolated were measured by thermistors inserted in the adsorbent and registered by a chart recorder. Calibration was carried out by passing electrical current through a coil immersed in the centre of the adsorbent beds with the solvents percolating through them.

The estimates of the amount of adsorption were carried out by measuring the time taken for the heat evolution to reach its maximum value, doubling it and assuming that during that time all the solute percolated through the adsorbent is adsorbed. The time interval in question usually corresponds to the major proportion of all the heat evolved during the adsorption process, i.e. the heat of adsorption without the adsorption "tail". The amounts of the solutes adsorbed calculated in this way are of course not very accurate, but give nevertheless some idea of the capacity of the solids to adsorb the solutes under the flow conditions, i.e. most probably non-equilibrium conditions. The level of adsorption was found to be 4.5g ± 1g of ethanol per 100g of the zeolites examined with the space velocity of solution flowing through the adsorbents being 30 volumes per hour.

Zeolite samples for use in the experiments were prepared as follows.

Gels of molar composition:-

$XSiO_2$ : $Al_2O_3$ : 5.09 NaOH : 333.6 $H_2O$ where X = 20 to 50 were prepared and crystallised for 72 h at 170° ± 1°C. A typical preparation of a high purity MFI zeolite using such a formulation is as follows:

3.66g (44.7 mmols) of sodium aluminate was added to a solution of 2.68g (67 mmols) of sodium hydroxide in 80.6g of deionised water and the mixture stirred for 30 min at room temperature during which time complete dissolution occurred (Solution A).

A second solution (B) was prepared meanwhile by adding 1.39g of aqueous ammonia to 85.8g of Ludox AS 40 colloidal silica. This solution was always prepared 25 min after the preparation of Solution A so that it was stirred for 5 min only.

As soon as the solutions A and B were ready Solution B was added to Solution A with rapid stirring. On mixing, a thick, opaque gel was formed which quickly became mobile with further stirring. After 10 min had elapsed 80 ml of the gel was placed in a clean 100 ml stainless steel pressure vessel which was immediately sealed and clamped to a rotating shaft in the crystallisation oven. The vessel was slowly rotated while being heated to 170°C, maintained at this temperature for 72 h and then rapidly cooled to room temperature.

When the pressure vessel was opened the product generally was a white solid suspended in an aqueous medium. The solid was rapidly separated from the mother liquor by filtration and washed with 400 ml of deionised water. The raw zeolite so obtained was dried to constant weight at 120°C.

Examples 1 to 3

Experiments were carried out as indicated above using MFI zeolites prepared as aforesaid with Si:Al atomic ratios of 17:1, 47:1. and 51:1 respectively. The heat of adsorption i.e. the heat evolved when water was displaced from the adsorbent by ethanol was measured and the amount of ethanol adsorbed was estimated. The results are given in the Table.

Comparative Test A

This is a comparative experiment not according to the invention.

An experiment was carried out as in Examples 1 to 3, but using silicalite as adsorbent (as disclosed in US 4 277 635).

Silicalite was prepared as follows:

60g of Ludox AS 40, an ammonium stabilised silica sol, was added, with stirring, to 60g of aqueous tetrapropylammonium hydroxide (5 per cent weight). After stirring for approximately 10 min, the mixture was placed in a 150ml stainless steel pressure vessel and heated to 170°C for 60 h under autogenous pressure. On cooling, the vessel was opened and the raw product separated from the mother liquor by filtration. The solid filter cake was then dried to constant weight at 120°C in an oven.

Before use, a sample of the silicalite was calcined in air at 500°C for 60 h. Powder X-ray diffraction analysis of the calcined sample showed it to be pure crystalline silicalite with no detectable amounts of amorphous material or other zeolite phases.

The results are given in the Table.

This shows that the estimated amount of ethanol adsorbed was comparable with that adsorbed by silicalite, but that the heat of adsorption was much greater for the zeolites of the present invention than for silicalite indicating a greater degree of selectivity in the separation. The heat of adsorption was especially high for zeolites with Si:Al ratios of 47:1 and 51:1 indicating that these zeolites should be especially effective in the separation of ethanol.

TABLE

Adsorption of Alcohols from Aqueous Solutiions

| Example | Adsorbent | Silica: alumina molar ratio | Heat of Adsorption J/g | Estimated Amount of Ethanol Adsorption mg/g |
|---|---|---|---|---|
| | | | Ethanol | |
| Comp Test A | Silicalite | 500:1 | 2.88 | 40 |
| 1 | MFI($NH_3$)* | 17:1 | 8.78 | 35 |
| 2 | MFI($NH_3$)* | 47:1 | 13.044 | 53 |
| 3 | MFI($NH_3$)* | 51.1 | 12.12 | 35 |

* MFI ($NH_3$) denotes an MFI-type crystalline aluminosilicate crystallised from a gel containing ammonia as the base.

Claims:

1. A process for the separation of ethanol from an aqueous solution thereof which comprises bringing the aqueous solution into contact with a synthetic crystalline aluminosilicate zeolite which is a high silica zeolite wherein the molar ratio of silica to alumina is in the range from 12:1 to 300:1.

2. A process according to claim 1 wherein the molar ratio of silica to alumina in the zeolite is in the range from 40:1 to 60:1.

3. A process according to either claim 1 or claim 2 wherein the aqueous ethanol solution contains less than 70% by weight of ethanol.

4. A process according to claim 3 wherein the aqueous ethanol solution contains less than 10% by weight of ethanol.

5. A process according to any one of the preceding claims wherein the crystalline aluminosilicate zeolite is an MFI-type crystalline aluminosilicate.

6. A process according to claim 5 wherein the MFI-type crystalline aluminosilicate is crystallised from a gel containing an inorganic source of ammonium ions.

7. A process according to claim 6 wherein the inorganic source of ammonium ions is aqueous ammonia.

8. A process according to any one of the preceding claims wherein the zeolite is employed in the form of a countercurrent moving bed.

9. A process according to any one of the claims 1 to 7 wherein the zeolite is employed in a simulated moving bed countercurrent flow system.